# EUROPÄISCHE PATENTSCHRIFT

(11) **EP 0 470 983 B1**
(45) Veröffentlichungstag und Bekanntmachung des Hinweises auf die Patenterteilung: **10.11.1993**
(21) Anmeldenummer: 90906935.3
(22) Anmeldetag: 28.04.1990
(51) Int. Cl.: A61B 1/04, A61B 1/00

(54) **KUPPLUNG FÜR EINE ELEKTRONISCHE KAMERA UND EINEN LICHTLEITER**
COUPLING FOR AN ELECTRONIC CAMERA AND FOR AN OPTICAL FIBRE WAVEGUIDE
COUPLEUR POUR UNE CAMERA ELECTRONIQUE ET UN GUIDE DE LUMIERE

(30) Priorität: 05.05.1989 DE 3914825
(43) Veröffentlichungstag der Anmeldung: 19.02.1992
(73) Patentinhaber: AESCULAP AG, D-78501 Tuttlingen (DE)
(72) Erfinder: OERTMANN, Friedrich-Wilhelm, D-7200 Tuttlingen (DE); STALLFORTH, Harald, D-7200 Tuttlingen (DE)
(74) Vertreter: Hoeger, Stellrecht & Partner
(86) Internationale Anmeldenummer: EP9000690
(87) Internationale Veröffentlichungsnummer: WO9013254

(56) Entgegenhaltungen:
- EP-A- 280 397
- GB-A- 2 148 526
- US-A- 4 639 772

## Beschreibung

Die Erfindung betrifft eine Kupplung für eine elektronische Kamera, einen der Kamera Licht zuführenden Bildlichtleiter sowie für einen Beleuchtungslichtleiter, wobei die Kupplung ein erstes und ein zweites Teil aufweist, von denen das erste die elektronische Kamera trägt, an der eine aus dem ersten Teil austretende elektrische Signalleitung angeschlossen ist, während in das zweite Teil der Bildlichtleiter einmündet.

Bei der Verwendung elektronischer Festkörperkameras an medizinischen Endoskopen und dergleichen ist es erwünscht, zwischen Kamera und Endoskop beziehungsweise Bildlichtleiter eine lösbare Kupplung vorzusehen, beispielsweise um die Kamera wahlweise mit unterschiedlichen Bildlichtleitern verschiedener medizinischer Instrumente verbinden zu können.

Um die Handhabbarkeit der Instrumente zu verbessern, ist es weiterhin erwünscht, eine freie Drehbarkeit des Bildlichtleiters um die Längsachse der Kupplung zu erreichen.

Dies ist bei zentral angeordneter Kamera und zentral angeordnetem Bildlichtleiter einfach zu erreichen, indem die Drehachse durch Kamera und Bildlichtleiter verläuft.

Wenn jedoch zusätzlich in der Kupplung auch eine Verbindung zwischen einem Beleuchtungslicht zuführenden Beleuchtungslichtleiter und einem dieses Licht zusammen mit dem Bildlichtleiter dem zu beobachtenden Bild zuführenden Beleuchtungslichtleiter hergestellt werden soll, ergeben sich bei der freien Drehbarkeit derartiger Kupplungen Probleme. So ist beispielsweise aus der GB-A 2 148 526 eine gattungsgemäße Kupplung bekannt, bei der jedoch das Beleuchtungslicht über eine radiale Zuleitung in den Kupplungsteil eingeführt wird, in dem sich der Bildlichtleiter befindet. Damit wird die freie Drehbarkeit des Bildlichtleiters erheblich beeinträchtigt. Mit einer solchen Kupplung ist es nicht möglich, das Beleuchtungslicht über den die Kamera tragenden Kupplungsteil zuzuführen.

Es ist Aufgabe der Erfindung, mit möglichst einfachen konstruktiven Mitteln eine gattungsgemäße Kupplung so auszugestalten, daß ohne Beeinträchtigung der freien Drehbarkeit sowohl der Übergang zwischen Bildlichtleiter und Kamera als auch der Übergang zwischen den Beleuchtungslichtleitern in den beiden Teilen der Kupplung gewährleistet ist.

Diese Aufgabe wird bei einer Kupplung der eingangs beschriebenen Art erfindungsgemäß dadurch gelöst, daß das erste und das zweite Teil im gekoppelten Zustand der Kupplung um eine Drehachse gegeneinander frei verdrehbar sind, die durch die Längsachse der Kamera und die mit ihr ausgerichtete Längsachse des Bildlichtleiters definiert ist, und daß die Beleuchtungslichtleiter beider Teile die Kamera koaxial umgebend, in senkrecht zur Drehachse verlaufenden Stirnflächen der beiden Teile ringförmig enden, wobei die Stirnflächen beider Teile aneinander anliegen und die ringförmigen Endflächen der Beleuchtungslichtleiter beider Teile unabhängig von der jeweiligen Verdrehstellung der beiden Teile deckungsgleich aufeinanderliegen.

Durch die Ausbildung einer ringförmigen Endfläche in der Tennungsebene der Kupplung werden die ringförmigen Endflächen der Beleuchtungslichtleiter unabhängig von der jeweiligen Verdrehstellung der beiden Teile der Kupplung immer vollflächig aneinanderliegen und eine einwandfreie Übertragung des Beleuchtungslichtes ermöglichen. Irgendeine Beeinträchtigung der freien Drehbarkeit oder der Beleuchtung ist daher aufgrund der speziellen Konstruktion nicht zu erwarten.

Dabei kann insbesondere vorgesehen sein, daß die Kamera aus dem ersten Teil über dessen Stirnfläche hervorsteht und in eine Ausnehmung im zweiten Teil hineinragt. Dies führt insgesamt zu einer verkürzten Baulänge der Kupplung und gewährleistet eine einwandfreie Ausrichtung des Bildlichtleiters und der Kamera.

Bei einer bevorzugten Ausführungsform kann der Zwischenraum zwischen den beiden Stirnflächen der beiden Teile mit einem lichtdurchlässigen Medium ausgefüllt sein, dessen Brechungsindex größer als 1 ist, vorzugsweise zum Beispiel 1,5. Dadurch können Reflexionsverluste an den Faserenden der Beleuchtungslichtleiter und der Bildlichtleiter gesenkt werden, beispielsweise um etwa 10%.

Die nachfolgende Beschreibung einer bevorzugten Ausführungsform der Erfindung dient im Zusammenhang mit der Zeichnung der näheren Erläuterung. Es zeigen:
- Figur 1: eine schematische Längsschnittansicht einer Kupplung für eine elektronische Kamera und einen Bildlichtleiter und
- Figur 2:: eine Ansicht des ersten Teils der Kupplung in Richtung der Schnittlinie 2-2 in Figur 1.

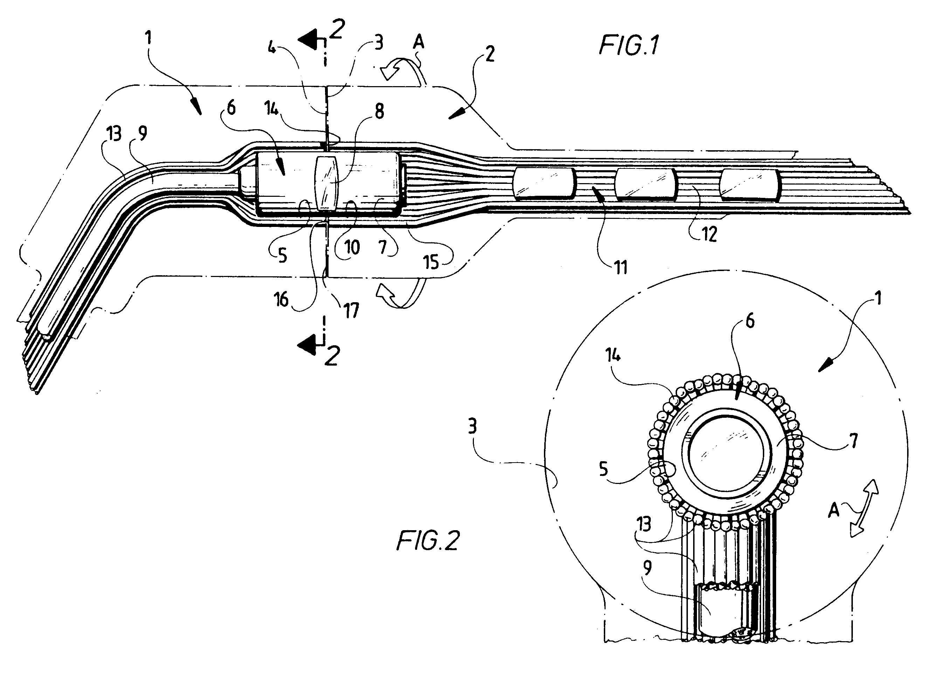

Die in der Zeichnung wiedergegebene Kupplung umfaßt ein erstes Teil 1 und ein zweites Teil 2, die jeweils in einer Stirnfläche 3 beziehungsweise 4 enden. Die beiden Teile 1 und 2 können unter Anlage der beiden Stirnflächen 3 und 4 axial unverschieblich aneinander festgelegt werden, beispielsweise durch einen in der Zeichnung nicht dargestellten Überwurfring, der zudem eine Lösbarkeit der beiden Teile voneinander gewährleistet. Um die senkrecht zu den Stirnflächen 3 und 4 verlaufende Längsachse sind die beiden Teile 1 und 2 frei gegeneinander verdrehbar, wie dies durch den Pfeil a in Figur 1 angedeutet wird.

Im ersten Teil 1 ist koaxial zur Drehachse in einem sich zur Stirnfläche 3 öffnenden zylindrischen Hohlraum 5 eine elektronische Kamera 6 angeordnet, die in an sich bekannter Weise ein zylinderförmiges Gehäuse 7 aufweist, in dem auf der Vorderseite eintretende Lichtstrahlung nach Durchlaufen eines optischen Elements 8 auf einen in der Zeichnung nicht dargestellten, lichtempfindlichen Halbleiter auftrifft und entsprechend dem auffallenden Licht elektrische Signale erzeugt, die über eine an der Rückseite des Gehäuses 7 austretende Signalleitung einer Verarbeitungseinheit zugeführt werden, die ebenfalls in der Zeichnung nicht dargestellt ist. Die Signalleitung 9 ist zunächst der Drehachse der beiden Teile der Kupplung folgend durch den ersten Teil 1 geführt und tritt dann unter einem Winkel gegenüber der Drehachse aus dem ersten Teil der Kupplung aus.

Das Gehäuse 7 ragt über die Stirnfläche 3 des ersten Teils 1 hervor und tritt in einen mit dem Hohlraum 5 ausgerichteten, ebenfalls zylindrischen Hohlraum 10 im zweiten Teil 2 der Kupplung ein. Dieser Hohlraum 10 verengt sich im Anschluß an das Kameragehäuse 7 und geht in einen rohrförmigen Bildlichtleiter 11 über, der beispielsweise Teil eines medizinischen Endoskopes sein kann. Dieser Bildlichtleiter 11 ist mit parallel verlaufenden Lichtleitfasern 12 ausgefüllt, die im Bereich vor der Kamera 6 entsprechend der Aufweitung des Hohlraums 10 aufgeweitet sind, so daß ihr Querschnitt dem Eintrittsquerschnitt der Kamera 6 entspricht.

Die Signalleitung 9 wird koaxial von einem Beleuchtungslichtleiter 13 umgeben, der aus einzelnen, unmittelbar nebeneinander angeordneten Lichtleitfasern besteht. Im Bereich des Hohlraumes 5, in dem sich die Kamera 6 befindet, weitet sich der koaxiale Beleuchtungslichtleiter 13 so auf, daß er die Kamera 6 koaxial umgibt und in der Stirnfläche 3 in Form eines den Hohlraum 5 koaxial umgebenden Ringes 14 endet.

Im zweiten Teil 2 der Kupplung ist ebenfalls ein den Bildlichtleiter 11 koaxial umgebender Beleuchtungslichtleiter 15 vorgesehen, der im Bereich des Hohlraumes 10 und der darin angeordneten Kamera 6 ebenfalls aufgeweitet ist und in der Stirnfläche 4 des Teiles 2 in Form eines Ringes 16 endet, wobei der Ring 14 und der Ring 16 der beiden Teile der Kupplung deckungsgleich sind und unmittelbar aneinanderliegen, so daß im Trennungsbereich der beiden Teile der Kupplung ein störungsfreier Übergang von Licht möglich ist, das durch den Beleuchtungslichtleiter 13 herangeführt und über den Beleuchtungslichtleiter 15 durch das Endoskop weitergeleitet wird. Der Lichtübergang im Bereich der Trennfläche der beiden Teile ist unabhängig von der relativen Winkelstellung der beiden Teile, das heißt diese sind frei gegeneinander verdrehbar, ohne daß dies auf die Übertragung vom Beleuchtungslichtleiter 13 zum Beleuchtungslichtleiter 15 einen Einfluß hätte.

Die Fasern der Beleuchtungslichtleiter können zu beiden Seiten der Trennebene der Kupplung selbstverständlich auch in anderer Weise geführt sein, das heißt sie müssen nicht den Bildlichtleiter beziehungsweise die Signalleitung koaxial umgeben. Es wäre beispielsweise möglich, die Fasern des Bildlichtleiters seitlich über einen separaten Anschlup aus den Teilen der Kupplung herauszuführen, so daß das Beleuchtungslicht über ein separates Lichtleitungskabel verläuft. Wesentlich ist lediglich, daß im Bereich der Trennebene zwischen den beiden Teilen der Kupplung eine koaxial zur Drehachse erfolgende ringförmige und deckungsgleiche Anordnung der Enden der Lichtleitfasern erfolgt.

Der Zwischenraum 17 zwischen den Stirnflächen 3 und 4 der beiden Teile 2 beziehungsweise 1 kann nach aupen abgedichtet und mit einem Medium gefüllt sein, welches lichtdurchlässig ist und einen Brechungsindex aufweist, der größer als 1 ist, vorzugsweise beispielsweise etwa 1,5. Dieses Medium kann eine Flüssigkeit sein. Durch die Verwendung eines solchen Mediums zwischen den beiden Stirnflächen und damit auch zwischen den Enden der Lichtleitfasern werden Reflexionsverluste an den Faserenden herabgesetzt, beispielsweise um etwa 10%.

Im Betrieb wird Beleuchtungslicht über Beleuchtungslichtleiter 13 und Beleuchtungslichtleiter 15 dem zu beobachtenden Bereich zugeleitet, das zurückfallende Licht wird über den Bildlichtleiter 11 der Kamera 6 zugeführt, die aufgrund der aufgenommenen Lichtstrahlung elektrische Signale erzeugt und diese über die Signalleitung 9 einer weiterverarbeitenden elektronischen Einheit zuführt.

Die beiden Teile der Kupplung können ohne weiteres in axialer Richtung voneinander gelöst werden, so daß ein Auswechseln oder eine Reinigung der entsprechenden Teile möglich ist.

## Patentansprüche

1. Kupplung für eine elektronische Kamera, einen der Kamera Licht zuführenden Bildlichtleiter sowie für einen Beleuchtungslichtleiter, wobei die Kupplung ein erstes und ein zweites Teil aufweist, von denen das erste die elektronische Kamera trägt, an der eine aus dem ersten Teil austretende elektrische Signalleitung angeschlossen ist, während in das zweite Teil der Bildlichtleiter einmündet, dadurch gekennzeichnet, daß das erste und das zweite Teil (1 beziehungsweise 2) im gekoppelten Zustand der Kupplung um eine Drehachse gegeneinander frei verdrehbar sind, die durch die Längsachse der Kamera (6) und die mit ihr ausgerichtete Längsachse des Bildlichtleiters (11) definiert ist, und daß die Beleuchtungslichtleiter (13, 15) beider Teile (1, 2) die Kamera (6) koaxial umgebend in senkrecht zur Drehachse verlaufenden Stirnflächen (3 beziehungsweise 4) der beiden Teile (1, 2) ringförmig enden, wobei die Stirnflächen (3 beziehungsweise 4) beider Teile (1, 2) aneinander anliegen und die ringförmigen Endflächen (14 beziehungsweise 16) der Beleuchtungslichtleiter (13 beziehungsweise 15) beider Teile (1, 2) unabhängig von der jeweiligen Verdrehstellung der beiden Teile deckungsgleich aufeinanderliegen.

2. Kupplung nach Anspruch 1, dadurch gekennzeichnet, daß die Kamera (6) aus dem ersten Teil (1) über dessen Stirnfläche (3) hervorsteht und in eine Ausnehmung (10) im zweiten Teil (2) hineinragt.

3. Kupplung nach Anspruch 1 oder 2, dadurch gekennzeichnet, daß der Zwischenraum (17) zwischen den beiden Stirnflächen (3, 4) der beiden Teile (1, 2) mit einem lichtdurchlässigen Medium ausgefüllt ist, dessen Brechungsindex größer als 1 ist.

## Claims

1. A coupling for an electronic camera, an image light guide supplying light to the camera, and an illumination light guide, the coupling having a first and a second part, the first of which carries the electronic camera to which an electrical signal lead emerging from the first part is connected, while the image light guide runs into the second part, characterised in that the first and the second part (1 and 2 respectively), in the coupled state of the coupling, are freely rotatable relative to one another about an axis of rotation which is defined by the longitudinal axis of the camera (6) and the longitudinal axis of the image light guide (11) aligned therewith, and in that the illumination light guides (13, 15) of the two parts (1, 2), surrounding the camera (6) coaxially, terminate in a circular shape in end faces (3 and 4 respectively) of the two parts (1, 2) extending perpendicularly to the axis of the rotation, wherein the end faces (3 and 4 respectively) of the two parts (1, 2) adjoin one another and the circular end surfaces (14 and 16 respectively) of the illumination light guides (13 and 15 respectively) of the two parts (1, 2) mate coincidently, irrespective of the particular rotational position of the two parts.

2. A coupling according to claim 1, characterised in that the camera (6) protrudes from the first part (1) across the end face (3) thereof and projects into a recess (10) in the second part (2).

3. A coupling according to claim 1 or 2, characterised in that the gap (17) between the two end faces (3, 4) of the two parts (1, 2) is filled with a light-permeable medium whose refractive index is greater than 1.

## Revendications

1. Coupleur destiné à une caméra électronique, à un câble fibres optiques d'image acheminant la lumière à la caméra ainsi qu'à un câble fibres optiques d'éclairage, le coupleur comportant une première et une seconde partie dont la première porte la caméra électronique à laquelle est connecté un conducteur électrique d'acheminement de signaux sortant de la première partie, alors que le câble fibres optiques d'image débouche dans la seconde partie, caractérisé en ce que la première et la seconde partie (1 et 2) lorsque le coupleur est à l'état couplé, peuvent tourner librement l'une par rapport à l'autre autour d'un axe de rotation qui est défini par l'axe longitudinal de la caméra (6) et l'axe longitudinal du câble fibres optiques d'image (11) qui est aligné avec celui-ci et en ce que les câbles fibres optiques d'éclairage (13, 15) des deux parties (1, 2) entourant coaxialement la caméra (6) se terminent annulairement par des faces frontales (3 et 4), perpendiculaires à l'axe de rotation, pour les deux parties (1, 2), les faces frontales (3 et 4) des deux parties (1, 2) étant accolées et les faces terminales annulaires (14 et 16) des câbles fibres optiques d'éclairage (13 et 15) des deux parties (1, 2) étant superposées en convergence, indépendamment de la position de rotation mutuelle des deux parties.

2. Coupleur selon la revendication 1, caractérisé en ce que la caméra (6) fait saillie hors de la première partie (1) au-delà de sa face frontale (3) et s'encastre dans un évidement (10) ménagé dans la seconde partie (2).

3. Coupleur selon la revendication 1 ou 2, caractérisé en ce que l'espace (17) entre les deux faces frontales (3, 4) des deux parties (1, 2) est empli d'un milieu transparent dont l'indice de réfraction est supérieur à 1.
